(19) Europäisches Patentamt
European Patent Office
Office européen des brevets

(11) Veröffentlichungsnummer: **0 075 855**
**B1**

# (12) EUROPÄISCHE PATENTSCHRIFT

(45) Veröffentlichungstag der Patentschrift: **27.12.85**

(21) Anmeldenummer: **82108758.2**

(22) Anmeldetag: **22.09.82**

(51) Int. Cl.⁴: **C 07 D 243/16**, C 07 D 487/04, A 61 K 31/55 // C07D245/06, (C07D487/04, 243:00, 203:00)

**(54) 7-Brom-5-(2-halogenphenyl)-1H-2,3-dihydro-1,4-benzodiazepin-Verbindungen sowie Verfahren und Zwischenprodukte zu ihrer Herstellung und diese Verbindungen enthaltende Arzneimittel.**

(30) Priorität: **30.09.81 DE 3138769**

(43) Veröffentlichungstag der Anmeldung: **06.04.83 Patentblatt 83/14**

(45) Bekanntmachung des Hinweises auf die Patenterteilung: **27.12.85 Patentblatt 85/52**

(84) Benannte Vertragsstaaten: **AT BE CH DE FR GB IT LI LU NL SE**

(56) Entgegenhaltungen:
**DE - A - 2 221 558**
**DE - A - 2 353 160**
**DE - A - 2 520 937**

(73) Patentinhaber: **Kali-Chemie Pharma GmbH, Hans-Böckler-Allee 20 Postfach 220, D-3000 Hannover 1 (DE)**

(72) Erfinder: **Zeugner, Horst, Dr., Dipl.-Chem., Havenweg 10, D-3000 Hannover 73 (DE)**
Erfinder: **Ruhland, Michael, Dr., Dipl.-Biol., Bachstelzenweg 14, D-3000 Hannover 61 (DE)**
Erfinder: **Liepmann, Hans, Dr., Dipl.-Chem., Auf dem Emmerberg 17, D-3000 Hannover 1 (DE)**
Erfinder: **Milkowski, Wolfgang, Dr.-Dipl.-Chem., Fasanenweg 13, D-3167 Burgdorf (DE)**
Erfinder: **Müsch, Herbert, Dr., Dipl.-Biol., Am Schönen Hoope 16, D-3015 Wennigsen 5 (DE)**

(74) Vertreter: **Lauer, Dieter, Dr., c/o Kali-Chemie Aktiengesellschaft Postfach 220, D-3000 Hannover 1 (DE)**

**EP 0 075 855 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 7-Brom-5-(2-halogenphenyl)-1H-2,3-dihydro-1,4-benzodiazepin-Verbindungen der allgemeinen Formel I

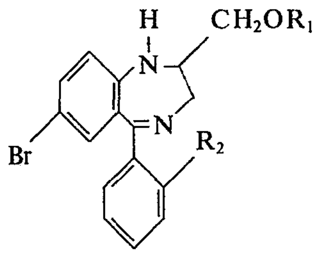

(I)

worin $R_1$ Wasserstoff, Alkyl mit 1—3 Kohlenstoffatomen oder Alkanoyl mit 2—4 Kohlenstoffatomen bedeutet, und $R_2$ Halogen bedeutet, und deren Säureadditionssalze, sowie diese Verbindungen enthaltende pharmazeutische Zubereitungen und Verfahren und Zwischenprodukte zur Herstellung dieser Verbindungen. Vorzugsweise stellt $R_1$ Wasserstoff oder Alkyl mit 1—2 Kohlenstoffatomen dar. Der Halogensubstituent $R_2$ steht insbesondere für Fluor, Chlor oder Brom, vorzugsweise Chlor.

In den deutschen Offenlegungsschriften 22 21 558 und 23 53 160 werden in 2-Stellung einen substituierten Methylrest tragende 5-Phenyl-1H-2,3-dihydro-1,4-benzodiazepin-Derivate beschrieben, welche das Zentralnervensystem beeinflussende, insbesondere antikonvulsive und tranquilisierende sowie sedierende und muskelrelaxierende Eigenschaften zeigen.

Die erfindungsgemäßen neuen Verbindungen fallen unter die in den genannten Offenlegungsschriften angegebenen allgemeinen Formeln, sind jedoch in diesen Offenlegungsschriften nicht beschrieben oder genannt worden.

In dem deutschen Patent 25 20 957 werden ebenfalls unter die allgemeinen Formeln der vorgehend genannnten Offenlegungsschriften fallende 1-Methyl-7-brom-2-alkoxymethyl-5-(2-halogenphenyl)-1H-2,3-dihydro-1,4-benzodiazepin-Verbindungen beansprucht. Aus diesem deutschen Patent ist bekannt, daß durch die Einführung eines Bromsubstituenten in 7-Stellung des 1-Methyl-5-phenyl-1,4-benzodiazepin-Gerüstes ein besonders günstiges Wirkungsprofil erreicht wird. So zeichnet sich die in dem deutschen Patent beanspruchte bevorzugte Gruppe von 7-Brom-1-methyl-2-alkoxymethyl-5-(2-halogenphenyl)-1H-2,3-dihydro-1,4-benzodiazepinen gegenüber den in den genannten deutschen Offenlegungsschriften aufgeführten Verbindungen, z. B. den zu den 7-Brom-Verbindungen analogen 7-Chlor-Verbindungen, aus durch einen deutlich verbesserten Dosisabstand zwischen einerseits antikonvulsiven, anxiolytisch und antiaggressiven Wirkungskomponenten und andererseits sedierenden und muskelrelaxierenden Wirkungskomponenten, welche zu unerwünschten Nebenwirkungen führen.

Der Erfindung liegt die Aufgabe zugrunde, neue anxiolytisch antiaggressiv wirksame 5-Phenyl-1H-2,3-dihydro-1,4-benzodiazepin-Derivate mit verbessertem Wirkungsprofil zu entwickeln.

Es wurde nun überraschend gefunden, daß die erfindungsgemäßen neuen in 1-Stellung unsubstituierten 2-Hydroxymethyl-, 2-Alkanoyloxymethyl- und 2-Alkoxymethyl-7-brom-5-(2-halogenphenyl)-1H-2,3-dihydro-1,4-benzodiazepine wertvolle psychopharmakologische Eigenschaften besitzen und eine ausgeprägte anxiolytisch-antiaggressive Wirkung entfalten und sich von den analogen 1-Methyl-2-hydroxymethyl- bzw. 1-Methyl-2-alkoxymethyl-7-brom-5-(2-halogenphenyl)-1H-2,3-dihydro-1,4-benzodiazepinen durch ein wesentlich günstigeres Wirkungsprofil unterscheiden.

So zeichnen sich die neuen in 1-Stellung unsubstituierten Verbindungen gegenüber analogen 1-Methyl-Verbindungen neben einer verbesserten antiaggressiven anxiolytischen Wirkung dadurch aus, daß die die Muskelkoordination und den Muskeltonus negativ beeinflussenden Wirkungskomponenten ganz wesentlich verringert sind. So liegen die wirksamen Dosen für eine Störung der Muskelkoordination bzw. des Muskeltonus für die erfindungsgemäßen in 1-Stellung unsubstituierten Verbindungen etwa doppelt bis dreifach so hoch wie für die entsprechenden 1-Methyl-Verbindungen. Die antiaggressiven anxiolytischen Wirkungen der erfindungsgemäßen Verbindungen zeigen sich dagegen schon bei Dosen, welche nur etwa halb so hoch sind als die Dosen, welche bei den entsprechenden 1-Methyl-Verbindungen benötigt werden. Somit wird eine Vervielfältigung des Abstandes zwischen wirksamer Dosis und unerwünschte Nebenwirkungen auslösender Dosis erzielt. Dieses günstige Wirkungsspektrum ist von großer Wichtigkeit für die Verwendung der Verbindungen für die ambulante Behandlung von psychisch kranken Menschen.

Das überraschend günstige Wirkungsprofil der erfindungsgemäßen in 1-Stellung unsubstituierten Verbindungen im Vergleich zu entsprechenden 1-Methyl-Verbindungen ist aus den Ergebnissen der nachstehend beschriebenen pharmakologischen Standardtests an Mäusen ersichtlich. Neben den erfindungsgemäßen Verbindungen und den dazu analogen 1-Methyl-Verbindungen wurde als Vergleichssubstanz auch Diazepam (Handelsprodukt: Valium®) mitgetestet. In der nachfolgenden Tabelle

werden bei den Testergebnissen die wirksamen Dosen für Diazepam = 1 gesetzt und die wirksamen Dosen der anderen Verbindungen als Vielfaches der Diazepamdosis angegeben.

Beschreibung der pharmakologischen Untersuchungsmethoden

1. Akute Toxizität.
   Die akute 7-Tage-Toxizität wird nach einmaliger Applikation per os an der weißen nüchternen NMRI-Maus bestimmt und die $LD_{50}$-Werte über EDV durch eine Probit-Analyse errechnet.
2. Prüfung auf anxiolytische und antiaggressive Wirksamkeit.
   Bestimmung der zur Hemmung der durch Isolation hervorgerufenen Aggressivität an der Maus wirksamen Dosis. Vor dem Versuch werden die Mäuse vier Wochen in strenger Isolation im Einzelkäfig gehalten. Nach dieser Zeit greifen die isoliert gehaltenen Mäuse nicht isoliert gehaltene Mäuse, die zugesetzt werden, spontan an. Die Prüfsubstanzen werden den isolierten Mäusen peroral verabreicht, und es wird nach 60 Minuten die Dosis bestimmt, die zu einer 50%igen Reduktion des aggressiven Verhaltens führt ($ED_{50}$). Die in dieser Versuchsanordnung (modifiziert nach Weischer und Opitz, Arch. int. Pharmacodyn. 195, 252 [1972]) erhaltenen Ergebnisse sind ein gutes Indiz für die angst-, streß- und spannungslösenden Eigenschaften der Verbindungen.
3. Prüfung der muskelkoordinationsstörenden Wirkungen.
   Das Muskelkoordinationsvermögen wird im Drehstabtest nach Blum (1973) an der Maus bestimmt.
   Für den Test werden nur männliche NMRI-Mäuse verwendet, welche sich 5 Minuten lang auf einem Drehstab mit einem Durchmesser von 4 cm bei einer Drehgeschwindigkeit von 12 Umdrehungen pro Minute halten können.
   Die Testsubstanzen werden den Tieren eine Stunde vor Testbeginn in Form einer 2%igen Suspension in Tyloselösung peroral verabreicht. Die Tiere werden je 2 × für eine Zeitspanne von 60 Sekunden auf den Drehstab gesetzt. Tiere, die sich nicht insgesamt 110 Sekunden auf dem drehenden Stab halten können, werden als in ihrer Muskelkoordination gestört angesehen. Als $ED_{50}$ wird eine Dosis angesehen, die bei 50% der Tiere eine Störung hervorruft.
4. Bestimmung der muskelrelaxierenden Wirkungen im Test de la Traction an der Maus (Arzneimittelforschung 17, 561 [1967]).
   In diesem Test wird die Beeinflussung des Muskeltonus durch die Prüfsubstanzen getestet. Die Prüfsubstanz wird Mäusen peroral verabreicht. Nach 120 Minuten werden die Mäuse mit den Vorderpfoten an einen dünnen waagerecht gespannten Draht gehängt. Als $ED_{50}$ gilt diejenige Dosis, bei der gerade die Hälfte der Tiere nicht innerhalb von 5 sec auch mit den Hinterpfoten den Draht berührt.

Die Ergebnisse der Testversuche sind in der folgenden Tabelle wiedergegeben. In den Spalten 2–4 ist jeweils der Quotient aus der $ED_{50}$ der betreffenden Testsubstanz dividiert durch den $ED_{50}$-Wert des Diazepam angegeben, d. h. die wirksame Diazepamdosis wurde = 1 gesetzt und die wirksamen Dosen der anderen Prüfsubstanzen als Vielfache davon angegeben.

In den Spalten 5 und 6 der Tabelle sind die Quotienten, die sich aus der die Muskelkoordination beeinträchtigenden bzw. der den Muskeltonus beeinträchtigenden Dosis und der anxiolytisch wirksamen Dosis ergeben, angegeben. Diese Quotienten zeigen, daß die Dosen, in denen negative muskulotrope Eigenschaften der erfindungsgemäßen Verbindungen zur Wirkung kommen, um ein Vielfaches höher liegen als die anxiolytisch-antiaggressiv wirksamen Dosen und daß der Abstand zwischen beiden Dosen bei den erfindungsgemäßen Verbindungen wesentlich höher ist als bei den dazu analogen 1-Methyl-Verbindungen.

Getestet wurden die Verbindungen der Formel A

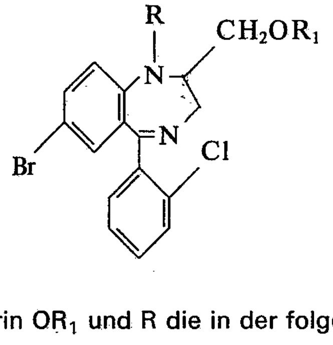

(A)

worin $OR_1$ und R die in der folgenden Tabelle angegebenen Bedeutungen besitzen.

| Testsubstanz der Formel A | 1 Akute Toxicität $LD_{50}$ mg/kg p. o. Maus | 2 Hemmung der durch Isolation erzeugten Aggression*) | 3 Störung der Muskelkoordination im Drehstabtest*) | 4 Störung des Muskeltonus im Test de la Traction*) | 5 Quotient $\frac{3}{2}$ | 6 Quotient $\frac{4}{2}$ |
|---|---|---|---|---|---|---|
| Substanz Beispiel 2 ($OR_1=OCH_3$, R=H) | 1330 | 0,59 | 3,17 | 32,0 | 5,38 | 54,2 |
| 1-$CH_3$ analoge Vergleichssubstanz ($OR_1=OCH_3$, $R=CH_3$) | >1470 | 1,17 | 1,30 | 8,63 | 1,11 | 7,38 |
| Substanz Beispiel 4 ($OR_1=OH$, R=H) | >1370 | 0,26 | 2,23 | 42,6 | 8,60 | 164 |
| 1-$CH_3$ analoge Vergleichssubstanz ($OR_1=OH$, $R=CH_3$) | >1470 | 0,65 | 0,87 | 25,2 | 1,3 | 38,8 |
| Diazepam | 850 | 1 | 1 | 1 | 1 | 1 |

*$ED_{50}$ mg/kg Testsubstanz: $ED_{50}$ mg/kg Diazepam.

Ferner wurde gefunden, daß im Gegensatz zu anderen bisher bekannten pharmakologisch aktiven Benzodiazepinen die erfindungsgemäßen Verbindungen, welche eine freie NH-Funktion in 1-Stellung des Ringgerüstes tragen, gegebenenfalls nach Freisetzung der OH-Gruppe aus einer allfälligen Acyloxy- oder Alkoxyseitenkette, direkt mit Glukuronsäure konjugiert ausgeschieden werden können, ohne vorher eine weitergehende Metabolisierung zu durchlaufen. Das heißt, daß Stoffwechsel und insbesondere Leberenzyme durch Einnahme der erfindungsgemäßen Verbindungen weitaus weniger belastet werden als durch andere Benzodiazepinderivate, welche eine Metabolisierung vor der Ausscheidung erfordern. Dies ist insbesondere von Bedeutung bei einer Langzeittherapie, wie sie gerade bei der Behandlung psychisch Kranker oft angewandt werden muß. Durch die leichte Ausscheidbarkeit der Verbindungen ist die Gefahr von Kumulation selbst bei langdauernder Behandlung praktisch ausgeschlossen.

Erfindungsgemäß werden die Verbindungen der Formel I hergestellt, indem man 2-Halogenmethyl-7-brom-5-(2-halogenphenyl)-1H-2,3-dihydro-1,4-benzodiazepin-Verbindungen der allgemeinen Formel III

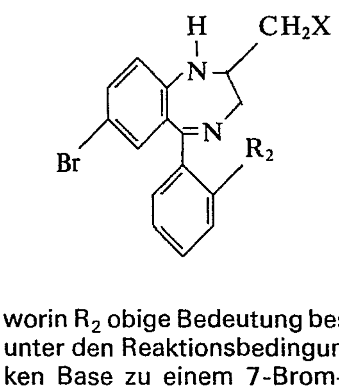

(III)

worin $R_2$ obige Bedeutung besitzt, und X Chlor, Brom oder Jod bedeutet, oder deren Gemische in einem unter den Reaktionsbedingungen inerten organischen Lösungsmittel durch Behandeln mit einer starken Base zu einem 7-Brom-1,2-methylen-5-(2-halogenphenyl)-1H-2,3-dihydro-1,4-benzodiazepin der allgemeinen Formel II

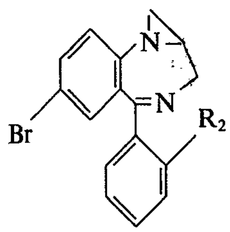

(II)

worin $R_2$ obige Bedeutung besitzt, cyclisiert, und anschließend zur Herstellung von Verbindungen der allgemeinen Formel Ia'

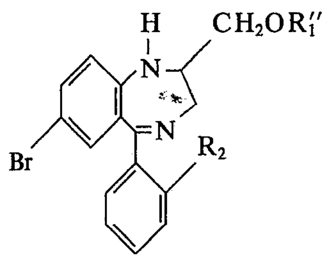

(Ia')

worin $R_2$ obige Bedeutung besitzt, und $R_1''$ Alkyl mit 1—3 Kohlenstoffatomen bedeutet, die Verbindung der Formel II in Gegenwart einer Lewissäure mit einem Alkohol der Formel IV'

$R_1''$—OH (IV')

worin $R_1''$ obige Bedeutung besitzt, umsetzt, oder zur Herstellung der Verbindung der allgemeinen Formel Ia''

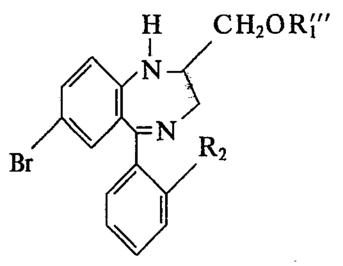

(Ia'')

worin $R_1'''$ Alkanoxyl mit 2—4 Kohlenstoffatomen bedeutet und $R_2$ obige Bedeutung besitzt, die Verbindung der Formel II in einem unter den Reaktionsbedingungen inerten organischen Lösungsmittel in Gegenwart einer Lewissäure mit einer niederen aliphatischen Carbonsäure der allgemeinen Formel IV''

$R_1'''$—OH (IV'')

worin $R_1'''$ obige Bedeutung besitzt, und die erhaltenen Ester der allgemeinen Formel Ia'' zu Verbindungen der allgemeinen Formel Ib

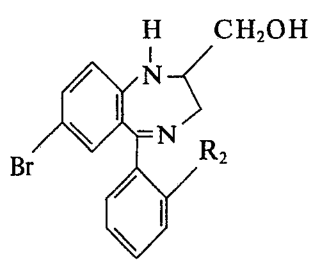

(Ib)

worin $R_2$ obige Bedeutung besitzt, hydrolisiert, und die erhaltenen Verbindungen der Formel I gewünschtenfalls in ihre Säureadditionssalze überführt oder die Säureadditionssalze in die freien Verbindungen der Formel I überführt.

Das erfindungsgemäße Verfahren ist in mehrfacher Hinsicht als überraschend anzusehen. Es ist zwar bekannt, daß Aziridin-Ringe aus aliphatischen beta-Halogenalkylaminen gebildet werden können. Es war jedoch nicht zu erwarten, daß die Überführung der Verbindungen der Formel III, welche phenyloge

Amidine darstellen (also nicht über eine basische Aminfunktion verfügen, sondern eine elektronenreiche Iminfunktion besitzen), unter Bildung einer 1,2-Methylenbrücke zu stabilen Verbindungen mit einem Ringsystem gelingt, in welchem ein Aziridinring an das Benzodiazepingerüst anelliert ist.

Ferner ist es überraschend, daß die anschließende Öffnung des Aziridinringes ausschließlich an der gewünschten Stelle stattfindet, d. h. ausschließlich zu dem in 2-Stellung substituierten 7-Ring des Benzodiazepingerüstes führt.

Die Cyclisierung der 2-Halogenmethylverbindungen der Formel III zu Verbindungen der Formel II wird zweckmäßigerweise in einem unter den Reaktionsbedingungen inerten Lösungsmittel in Gegenwart einer starken Base bei erhöhter Temperatur, beispielsweise bei Temperaturen zwischen 50 und 150°C, durchgeführt. Als starke Basen eignen sich beispielsweise niedere Alkalimetallalkoholate wie z. B. Natriummethylat, Natriumäthylat oder Natriumtertiärbutylat oder Alkalimetall- oder Erdalkalimetallhydride wie z. B. Natriumhydrid, Lithiumhydrid oder Calciumhydrid. Als inerte Lösungsmittel eignen sich beispielsweise niedere Alkohole, aromatische Kohlenwasserstoffe wie Toluol oder Xylol, Dimethylformamid oder Mischungen solcher Lösungsmittel. So sind beispielsweise bei Verwendung von Alkalimetallalkoholaten die entsprechenden Alkohole und bei Verwendung von Metallhydriden aromatische Kohlenwasserstoffe oder Dimethylformamid als Lösungsmittel besonders geeignet.

Die Öffnung des Aziridinringes der Verbindungen der Formel II erfolgt durch Umsetzung der Verbindungen der Formel II mit einem niederen Alkohol der Formel IV', das ist Methanol, Äthanol, n-Propanol oder Isopropanol, oder mit einer Säure der Formel IV'', vorzugsweise Essigsäure oder Propionsäure, in Gegenwart einer Lewissäure und eines Lösungsmittels. Als Lösungsmittel können im Falle der Umsetzung mit niederen Alkoholen diese Alkohole selbst dienen. Gewünschtenfalls können weitere unter den Reaktionsbedingungen inerte organische Lösungsmittel, beispielsweise halogenierte Kohlenwasserstoffe wie Methylenchlorid oder aromatische Kohlenwasserstoffe wie Toluol oder Xylol oder aliphatische oder aromatische Äther, beispielsweise Diäthyläther oder Tetrahydrofuran zugesetzt werden. Im Falle der Umsetzung mit einer niederen Carbonsäure ist es zweckmäßig, eines der vorgenannten Lösungsmittel zuzusetzen. Als Lewissäuren eignen sich insbesondere Borhalogenide wie Bortrichlorid oder vorzugsweise Bortrifluorid. Die Umsetzung kann bei Temperaturen zwischen 0 und 30°C, vorzugsweise bei Raumtemperatur, erfolgen.

Die bei der Umsetzung der Verbindungen der Formel II mit niederen aliphatischen Carbonsäuren erhaltenen Ester der Formel Ia'' können auf an sich bekannte Weise hydrolysiert werden. Zweckmäßigerweise werden die Ester einer alkalischen Hydrolyse unterworfen, beispielsweise mittels anorganischer Basen wie Alkalimetallhydroxiden oder -carbonaten, beispielsweise Natriumhydroxid, Kaliumhydroxid oder Natriumcarbonat. Die Umsetzung erfolgt zweckmäßigerweise bei erhöhter Temperatur, vorzugsweise Siedetemperatur des Reaktionsgemisches. Gewünschtenfalls können mit Wasser mischbare organische Lösungsmittel, vorzugsweise niedere Alkohole, wie Methanol oder Äthanol, zugesetzt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise Salze anorganischer Säuren, wie Chlorwasserstoffsäure, Bromwasserstoffsäure, Schwefelsäure oder Phosphorsäure, oder Salze organischer Säuren, wie beispielsweise Maleinsäure, Fumarsäure, Essigsäure, Benzoesäure, Methansulfonsäure, Cyclohexylaminosulfonsäure, Milchsäure, Weinsäure oder Phenylessigsäure.

Die Ausgangsverbindungen der Formel III können erhalten werden, indem man Verbindungen der Formel V

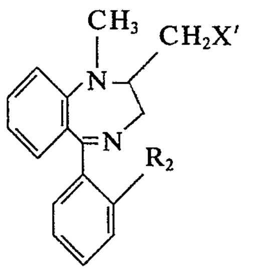

(V)

worin $R_2$ obige Bedeutung besitzt, und X' Chlor oder Brom bedeutet, auf an sich bekannte Weise durch Umsetzung mit Jodwasserstoff entmethyliert und die erhaltenen Verbindungen der Formel VI

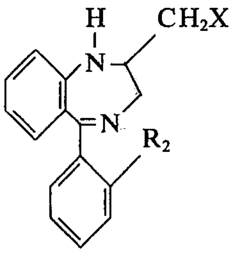

(VI)

worin $R_2$ und X obige Bedeutung besitzen, auf an sich bekannte Weise bromiert.

Die Verbindungen der Formel V können in bekannter Weise beispielsweise nach den in den deutschen Offenlegungsschriften 22 21 558 oder 23 53 187 oder dem deutschen Patent 25 20 937 oder dem deutschen Patent 25 20 937 beschriebenen Methoden ausgehend von $N_1$-Phenyl-$N_1$-methyl-$N_2$-(2-halogenphenyl)-2-hydroxy-1,3-diaminopropan durch Behandlung mit einem Phosphoroxidhalogenid, vorzugsweise Phosphoroxidchlorid, und anschließende Isomerisierung des entstehenden Cyclisierungsgemisches erhalten werden.

Die Entmethylierung der Verbindungen der Formel V mittels Jodwasserstoffsäure kann in an sich bekannter Weise erfolgen. Zweckmäßigerweise wird die Reaktion mit konzentrierter Jodwasserstoffsäure gegebenenfalls in einem unter den Reaktionsbedingungen inerten Lösungsmittel, beispielsweise einer niederen aliphatischen Carbonsäure, wie Essigsäure, bei Temperaturen zwischen 50 und 100°C durchgeführt.

Bei der Reaktion wird das Halogen in der Seitenkette teilweise durch Jod ersetzt, so daß ein Gemisch von Halogeniden der Formel VI entsteht. Dieses kann ohne weitere Auftrennung für die weiteren Reaktionen eingesetzt werden.

Die Bromierung der Verbindungen der Formel VI kann auf an sich bekannte Weise unter Verwendung von N-Bromsuccinimid in einem unter den Reaktionsbedingungen inerten Lösungsmittel, beispielsweise einem halogenierten Kohlenwasserstoff wie Methylenchlorid, erfolgen.

Aufgrund ihrer vorstehend beschriebenen pharmakologischen Eigenschaften stellen die erfindungsgemäßen neuen Verbindungen wertvolle Psychopharmaka dar.

Zur Verwendung als Arzneistoffe können sowohl die freien Basen als auch deren pharmakologisch annehmbaren Säureadditionssalze eingesetzt werden. Als Heilmittel können die Verbindungen der Formel I und ihre physiologisch verträglichen Säureadditionssalze zusammen mit üblichen pharmazeutischen Hilfsstoffen in galenischen Zubereitungen, wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe, wie z. B. Milchzucker, Stärke oder Talkum oder flüssiger Verdünnungsmittel, wie z. B. Wasser, fetten Ölen oder flüssigen Paraffinen. Diese pharmazeutischen Zubereitungen können zwischen 1 bis 50 mg Aktivsubstanz pro Einzeldosis enthalten. Die verwendete Dosierung wird selbstverständlich der zu behandelnden Spezies und den individuellen Erfordernissen angepaßt werden.

Die nachfolgenden Beispiele erläutern die Herstellung der neuen Verbindungen. Sie sollen jedoch den Umfang der Erfindung in keiner Weise beschränken.

## Beispiel 1

### 7-Brom-1,2-methylen-5-(2-chlorphenyl)-1H-2,3-dihydro-1,4-benzodiazepin

A) 50 g N-Methyl-N-[2-hydroxy-3-(2-chlorbenzoylamino)-propyl]-anilin werden in 250 ml Phosphoroxidchlorid 2,5 Stunden unter Rückfluß gekocht. Dann wird das überschüssige Phosphoroxidchlorid in Vacuum weitgehend abdestilliert und der obige Rückstand auf ein Gemisch aus 300 g Eis und 300 ml Wasser gegossen. Darauf wird das Reaktionsprodukt 3 × mit je 200 ml Chloroform extrahiert. Die Chloroformphase wird mit 200 ml Wasser gewaschen und anschließend mit verdünnter Natronlauge (20%) bis zur alkalischen Reaktion geschüttelt. Die Chloroformphase wird mit Wasser neutral gewaschen, über Natriumsulfat getrocknet und nach dem filtrieren im Vacuum eingedampft. Zur weiteren Reinigung wird der Rückstand (45,1 g) in Äther gelöst und vom ausfallenden Niederschlag abfiltriert. Nach dem Abdestillieren des Äthers werden 35,8 g eines Gemisches aus 1-Methyl-3-chlor-6-(2-chlorphenyl)-1,2,3,4-tetrahydro-1,5-benzodiazocin und 1-Methyl-2-chlormethyl-5-(2-chlorphenyl)-2,3-dihydro-1H-1,4-benzodiazepin erhalten. Zur Isomerisierung der Benzodiazocinkomponente des Cyclisierungsgemisches wird dieses in 150 ml Tetrachloräthan gelöst und die Lösung 30 Minuten unter Rückfluß gekocht. Nach dem Abdestillieren des Lösungsmittels werden 35,2 g 1-Methyl-2-chlormethyl-5-(2-chlorphenyl)-2,3-dihydro-1H-1,4-benzodiazepin erhalten, welches ohne weitere Reinigung in die folgende Reaktion eingesetzt werden kann.

B) 50 g 1-Methyl-2 chlormethyl 5-(2-chlorphenyl)-1H-2,3-dihydro-1,4-benzodiazepin werden in 93 ml Essigsäure gelöst, die Lösung mit 150 ml Jodwasserstoffsäure (65%) versetzt und vier Stunden auf 80°C erwärmt. Dann wird die Reaktionslösung abgekühlt und auf 500 g Eis gegossen. Nach dem Schmelzen des Eises wird das Reaktionsprodukt mit Methylenchlorid extrahiert und die organische Phase mit eiskalter Natronlauge (10%) bis zur alkalischen Reaktion versetzt. Die Methylenchloridphase wird anschließend abgetrennt, neutral gewaschen, über Natriumsulfat getrocknet, filtriert und das Lösungsmittel im Vakuum entfernt. Es verbleiben als Rückstand 56 g öliges Rohprodukt, welches ein Gemisch von 80% 2-Chlormethyl-5-(2-chlorphenyl)-1H-2,3-dihydro-1,4-benzodiazepin und 20% 2-Jodmethyl-5-(2-chlorphenyl)-1H-2,3-dihydro-1,4-benzodiazepin darstellt.

C) 120 g eines wie vorstehend beschrieben erhaltenen Gemisches werden in 900 ml Methylenchlorid gelöst, die Lösung mit 58 g N-Bromsuccinimid versetzt und über Nacht bei Raumtemperatur gerührt. Die Reaktionslösung wird anschließend mit Wasser, dann mit 10%iger Natriumcarbonatlösung und wiederum mit Wasser gewaschen, über Natriumsulfat getrocknet, filtriert, und das Lösungsmittel abdestilliert. Als Rückstand werden 111 g Rohprodukt erhalten, welches eine Mischung aus etwa 80% 2-Chlormethyl-7-brom-5-(2-chlorphenyl)-1H-2,3-dihydro-1,4-benzodiazepin und 20% 2-Jodmethyl-7-brom-5-(2-chlorphenyl)-1H-2,3-dihydro-1,4-benzodiazepin enthält.

Zur Überführung in das Hydrochlorid wird dieses Rohprodukt in Aceton gelöst und mit einer Lösung von Chlorwasserstoffgas in Äther versetzt. Die ausgeschiedenen Kristalle werden abfiltriert und mehrmals aus Äthanol/Aceton umkristallisiert. Es werden 61 g 2-Chlormethyl-7-brom-5-(2-chlorphenyl)-1H-2,3-dihydro-1,4-benzodiazepin-hydrochlorid mit dem Schmelzpunkt 240 bis 243°C erhalten, welche noch 10% der analogen 2-Jodmethyl-Verbindung enthalten.

D 42 g dieses 7-Brom-2-chlormethyl-5-(2-chlorphenyl)-1H-2,3-dihydro-1,4-benzodiazepinhydrochlorid werden in 100 ml Methanol gelöst und mit einer Lösung von 9,6 g Natrium in 320 ml Methanol versetzt und das Reaktionsgemisch zwei Stunden unter Rückfluß gekocht. Nach dem Abkühlen wird die Reaktionsmischung auf 400 ml Wasser gegossen, das kristallin ausgeschiedene 7-Brom-1,2-methylen-5-(2-chlorphenyl)-1H-2,3-dihydro-1,4-benzodiazepin wird abfiltriert und aus Aceton umkristallisiert.

Schmelzpunkt 172 bis 174°C, Ausbeute 29,4 g.

## Beispiel 2

### 7-Brom-2-methoxymethyl-5-(2-chlorphenyl)-1H-2,3-dihydro-1,4-benzodiazepin

25 g 7-Brom-1,2-methylen-5-(2-chlorphenyl)-1H-2,3-dihydro-1,4-benzodiazepin werden in 270 ml Methanol gelöst und die Lösung wird unter Rühren und Kühlen bei 20 bis 30°C mit 52 ml Bortrifluorid-Ätherat versetzt. Das Reaktionsgemisch wird zwei Stunden bei Raumtemperatur gerührt und anschließend mit 500 ml Toluol verdünnt. Die Toluol-Lösung wird mit gesättigter Natriumbicarbonatlösung und dann mit Wasser gewaschen, über Natriumsulfat getrocknet, filtriert und das Lösungsmittel abdestilliert. Als Rückstand verbleiben 31 g rohes 7-Brom-2-methoxymethyl-5-(2-chlorphenyl)-1H-2,3-dihydro-1,4-benzodiazepin. Dieser Rückstand wird in Äther gelöst und mit einer Lösung von Chlorwasserstoffgas in Äther versetzt. Die ausgeschiedenen Hydrochloridkristalle werden abfiltriert und mit einer Mischung aus Aceton und wenig Äthanol dreimal ausgekocht. Es werden 42,9 g 7-Brom-2-methoxymethyl-5-(2-chlorphenyl)-1H-2,3-dihydro-1,4-benzodiazepin-hydrochlorid erhalten. Schmelzpunkt 234 bis 236°C.

## Beispiel 3

### 7-Brom-2-acetoxymethyl-5-(2-chlorphenyl)-1H-2,3-dihydro-1,4-benzodiazepin

55 g 7-Brom-1,2-methylen-5-(2-chlorphenyl)-1H-2,3-dihydro-1,4-benzodiazepin werden in 450 ml Methylenchlorid gelöst, und zu der Lösung werden 180 ml Essigsäure gegeben. Dann werden unter Kühlung auf 0°C 110 ml Bortrifluorid-Ätherat langsam zugetropft. Die Reaktionsmischung wird noch eine Stunde bei Raumtemperatur gerührt und anschließend auf eine Mischung aus Eis und Wasser gegossen. Die organische Phase wird abgetrennt und mit Wasser, dann mit verdünnter Natronlauge (10%) und wiederum mit Wasser gewaschen, über Natriumsulfat getrocknet, filtriert und eingedampft. Als Rückstand verbleiben 68 g rohes 7-Brom-2-acetoxymethyl-5-(2-chlorphenyl)-1H-2,3-dihydro-1,4-benzodiazepin erhalten.

Beispiel 4

7-Brom-2-hydroxymethyl-5-(2-chlorphenyl)-1H-2,3-dihydro-1,4-benzodiazepin

68 g 7-Brom-2-acetoxymethylen-5-(2-chlorphenyl)-1H-2,3-dihydro-1,4-benzodiazepin werden ohne weitere Reinigung mit 640 ml einer Lösung von Kaliumhydroxid in Methanol (15%ig) eine Stunde unter Rückfluß gekocht. Anschließend wird das Lösungsmittel abdestilliert und der Rückstand in Wasser aufgenommen und mit Äther extrahiert. Nach üblicher Aufarbeitung der Ätherphase werden 53 g rohes 7-Brom-2-hydroxymethyl-5-(2-chlorphenyl)-1H-2,3-dihydro-1,4-benzodiazepin erhalten. Zur Überführung in das Hydrochlorid wird eine ätherische Lösung dieses Rohalkohols mit einer Lösung von Chlorwasserstoffgas in Äther versetzt und das ausgefallene 7-Brom-2-hydroxymethyl-5-(2-chlorphenyl)-1H-2,3-dihydro-1,4-benzodiazepin-hydrochlorid isoliert. Schmelzpunkt 228–232°C, Ausbeute 26,1 g.

Beispiel I

Tabletten

Man stellt Tabletten in folgender Zusammensetzung pro Tablette her:

| | |
|---|---|
| 7-Brom-2-hydroxymethyl-5-(2-chlorphenyl)-1H-2,3-dihydro-1,4-benzodiazepin-hydrochlorid | 25 mg |
| Maisstärke | 60 mg |
| Milchzucker | 130 mg |
| Gelatine (10%ige Lösung) | 6 mg |

Der Wirkstoff, die Maisstärke und der Milchzucker werden mit der 10%igen Gelatinelösung eingedickt. Die Paste wird zerkleinert und das entstandene Granulat wird auf ein geeignetes Blech gebracht und bei 45°C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

**Patentansprüche für die Vertragsstaaten: BE, CH, DE, FR, GB, IT, LI, LU, NL, SE**

1. 7-Brom-5-(2-halogenphenyl)-1H-2,3-dihydro-1,4-benzodiazepin-Verbindungen der allgemeinen Formel I

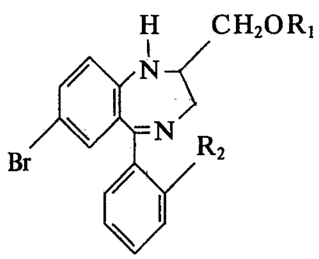

(I)

worin $R_1$ Wasserstoff, Alkyl mit 1–3 Kohlenstoffatomen oder Alkanoyl mit 2–4 Kohlenstoffatomen bedeutet, und $R_2$ Halogen bedeutet, und deren Säureadditionssalze.

2. Verbindungen der Formel I gemäß Anspruch 1, worin $R_2$ Chlor bedeutet.

3. Verbindungen der Formel I gemäß Anspruch 1 oder 2; worin $R_1$ Wasserstoff, Methyl oder Äthyl bedeutet.

4. 7-Brom-1,2-methylen-5-(2-halogenphenyl)-1H-2,3-dihydro-1,4-benzodiazepine der allgemeinen Formel II

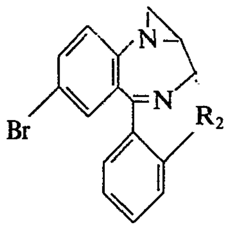

(II)

worin $R_2$ Halogen bedeutet, und deren Säureadditionssalze.

5. Heilmittel enthaltend Verbindungen der allgemeinen Formel I gemäß Anspruch 1 oder deren pharmakologisch verträgliche Säureadditionssalze und übliche pharmazeutische Trägerstoffe.

6. Verfahren zur Herstellung von 7-Brom-5-(2-halogenphenyl)-1H-2,3-dihydro-1,4-benzodiazepin-Verbindungen der allgemeinen Formel I

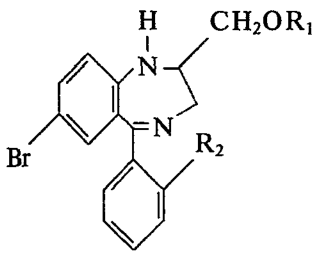

(I)

worin $R_1$ Wasserstoff, Alkyl mit 1—3 Kohlenstoffatomen oder Alkanoyl mit 2—4 Kohlenstoffatomen bedeutet, und $R_2$ Halogen bedeutet, und deren Säureadditionssalze, dadurch gekennzeichnet, daß man 2-Halogenmethyl-7-brom-5-(2-halogenphenyl)-1H-2,3-dihydro-1,4-benzodiazepin Verbindungen der allgemeinen Formel III

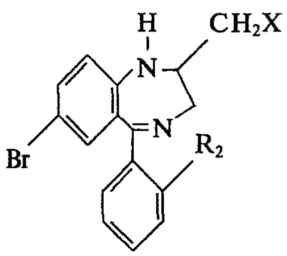

(III)

worin $R_2$ obige Bedeutung besitzt, und X Chlor, Brom oder Jod bedeutet, oder deren Gemische in einem unter den Reaktionsbedingungen inerten organischen Lösungsmittel durch Behandeln mit einer starken Base zu 7-Brom-1,2-methylen-5-(2-halogenphenyl)-1H-2,3-dihydro-1,4-benzodiazepinen der allgemeinen Formel II

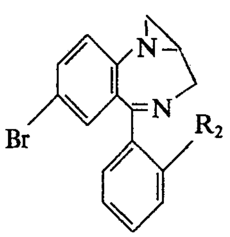

(II)

worin $R_2$ obige Bedeutung besitzt, cyclisiert, und die Verbindungen der Formel II in Gegenwart einer Lewissäure mit Verbindungen der Formel IV

$$R_1'—OH \qquad (IV)$$

worin $R_1'$ Alkyl mit 1—3 Kohlenstoffatomen oder Alkanoyl mit 2—4 Kohlenstoffatomen bedeutet, umsetzt zu Verbindungen der Formel Ia

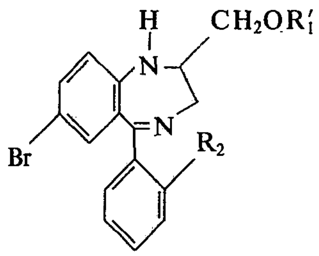

(Ia)

worin $R_1'$ und $R_2$ obige Bedeutung besitzen, und erhaltene Ester der allgemeinen Formel Ia, worin $R_1'$ Alkanoyl mit 2–4 Kohlenstoffatomen bedeutet, gewünschtenfalls zu Verbindungen der allgemeinen Formel Ib

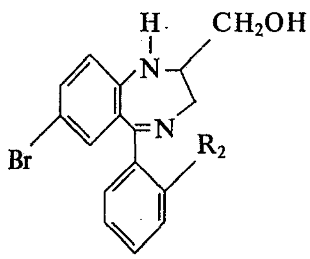

(Ib)

worin $R_2$ obige Bedeutung besitzt, hydrolysiert, und die erhaltenen Verbindungen der Formel I gewünschtenfalls in ihre Säureadditionssalze überführt oder die Säureadditionssalze in die freien Verbindungen der Formel I überführt.

**Patentansprüche für den Vertragsstaat: AT**

1. Verfahren zur Herstellung von 7-Brom-5-(2-halogenphenyl)-1H-2,3-dihydro-1,4-benzodiazepin-Verbindungen der allgemeinen Formel I

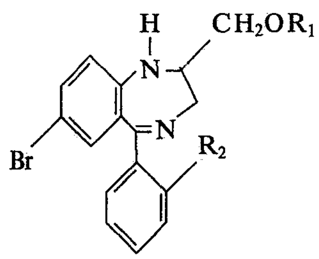

(I)

worin $R_1$ Wasserstoff oder Alkyl mit 1–3 Kohlenstoffatomen oder Alkanoyl mit 2–4 Kohlenstoffatomen bedeutet, und $R_2$ Halogen bedeutet, und deren Säureadditionssalze, dadurch gekennzeichnet, daß man 2-Halogenmethyl-7-brom-5-(2-halogenphenyl)-1H-2,3-dihydro-1,4-benzodiazepin-Verbindungen der allgemeinen Formel III

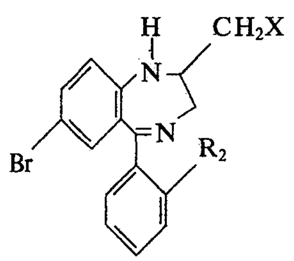

(III)

worin $R_2$ obige Bedeutung besitzt, und X Chlor, Brom oder Jod bedeutet, oder deren Gemische in einem unter den Reaktionsbedingungen inerten organischen Lösungsmittel durch Behandeln mit einer starken Base zu 7-Brom-1,2-methylen-5-(2-halogenphenyl)-1H-2,3-dihydro-1,4-benzodiazepinen der Formel II

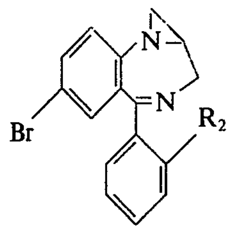

(II)

worin $R_2$ obige Bedeutung besitzt, cyclisiert, und die Verbindungen der Formel II in Gegenwart einer Lewissäure mit Verbindungen der Formel IV

$$R_1'\text{—OH} \qquad \text{(IV)}$$

worin $R_1'$ Alkyl mit 1–3 Kohlenstoffatomen oder Alkanoyl mit 2–4 Kohlenstoffatomen bedeutet, umsetzt zu Verbindungen der Formel Ia

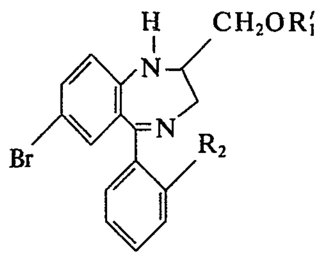

(Ia)

worin $R_1'$ und $R_2$ obige Bedeutung besitzen, und erhaltene Ester der allgemeinen Formel Ia, worin $R_1'$ Alkanoyl mit 2–4 Kohlenstoffatomen bedeutet, gewünschtenfalls zu Verbindungen der allgemeinen Formel Ib

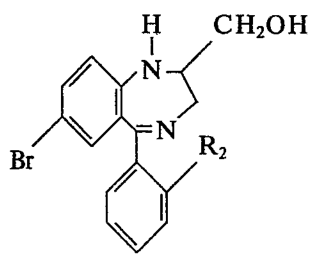

(Ib)

worin $R_2$ obige Bedeutung besitzt, hydrolysiert, und die erhaltenen Verbindungen der Formel I gewünschtenfalls in ihre Säureadditionssalze überführt oder die Säureadditionssalze in die freien Verbindungen der Formel I überführt.

2. Verfahren nach Anspruch 1, dadurch gekennzeichnet, daß solche Verbindungen der Formel I hergestellt werden, worin $R_2$ Chlor bedeutet.

3. Verfahren nach Anspruch 1 oder 2, dadurch gekennzeichnet, daß solche Verbindungen der Formel I hergestellt werden, worin $R_1$ Wasserstoff, Methyl oder Äthyl bedeutet.

4. Verfahren zur Herstellung von 7-Brom-1,2-methylen-5-(2-halogenphenyl)-1H-2,3-dihydro-1,4-benzodiazepinen der allgemeinen Formel II

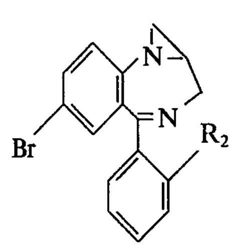

(II)

worin $R_2$ Halogen bedeutet, und deren Säureadditionssalze, dadurch gekennzeichnet, daß man 2-Halogenmethyl-7-brom-5-(2-halogenphenyl)-1H-2,3-dihydro-1,4-benzodiazepin-Verbindungen der allgemeinen Formel III

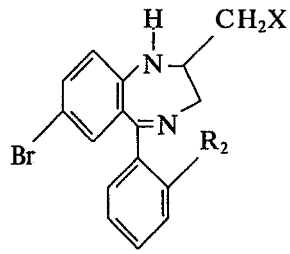

(III)

worin $R_2$ obige Bedeutung besitzt, und X Chlor, Brom oder Jod bedeutet, oder deren Gemische in einem unter den Reaktionsbedingungen inerten organischen Lösungsmittel durch Behandeln mit einer starken Base cyclisiert.

**Claims for the Contracting States: BE, CH, DE, FR, GB, IT, LI, LU, NL, SE**

1. 7-Bromo-5-(2-halophenyl)-1H-2,3-dihydro-1,4-benzodiazepines of the general formula I

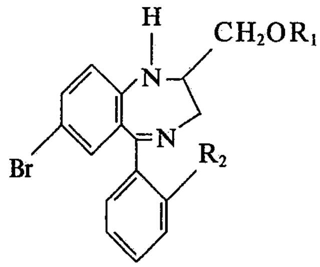

(I)

in which $R_1$ is hydrogen, alkyl with 1—3 carbon atoms or alkanoyl with 2—4 carbon atoms, and $R_2$ is halogen, and their acid addition salts.

2. Compounds of formula I according to Claim 1, in which $R_2$ is chlorine.

3. Compounds of formula I according to Claim 1 or 2, in which $R_1$ is hydrogen, methyl or ethyl.

4. 7-Bromo-1,2-methylene-5-(2-halophenyl)-1H-2,3-dihydro-1,4-benzodiazepines of the general formula II

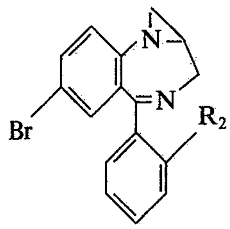

(II)

in which $R_2$ is halogen, and their acid addition salts.

5. Medicaments containing compounds of the general formula I according to Claim 1 or their pharmacologically acceptable acid addition salts and conventional pharmaceutical carrier substances.

6. Method for the preparation of 7-bromo-5-(2-halophenyl)-1H-2,3-dihydro-1,4-benzodiazepine compounds of the general formula I

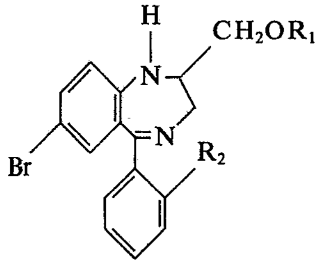

(I)

in which $R_1$ is hydrogen or alkyl with 1–3 carbon atoms or alkanoyl with 2–4 carbon atoms, and $R_2$ is halogen, and their acid addition salts, characterised in that 2-halomethyl-7-bromo-5-(2-halophenyl)-1H-2,3-dihydro-1,4-benzodiazepine compounds of the general formula III

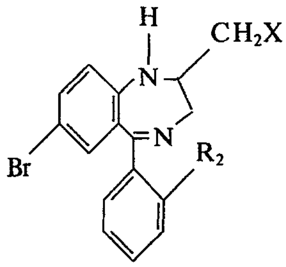

(III)

in which $R_2$ has the above meaning, and X is chlorine, bromine or iodine, or mixtures thereof, are cyclized in an organic solvent which is inert under the reaction conditions by treatment with a strong base to form 7-bromo-1,2-methylene-5-(2-halophenyl)-1H-2,3-dihydro-1,4-benzodiazepines of formula II

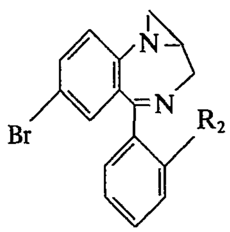

(II)

in which $R_2$ has the above meaning, and the compounds of formula II are reacted in the presence of a Lewis acid with compounds of formula IV

$$R_1'—OH \qquad (IV)$$

in which $R_1'$ is alkyl with 1–3 carbon atoms or alkanoyl with 2–4 carbon atoms to form compounds of formula Ia

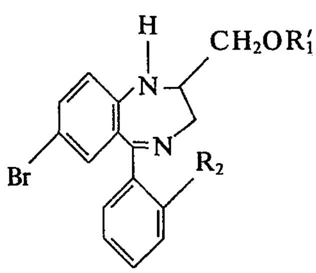

(Ia)

in which $R_1$ and $R_2$ have the above meanings, and resulting esters of the general formula Ia, in which $R_1'$ is alkanoyl with 2–4 carbon atoms optionally are hydrolysed to form compounds of the general formula Ib

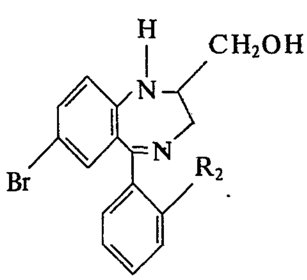

(Ib)

in which $R_2$ has the above meaning, and the resulting compounds of formula I optionally are converted into their acid addition salts or the acid addition salts are converted into the free compounds of formula I.

**Claims for the Contracting State: AT**

1. Method for the preparation of 7-bromo-5-(2-halophenyl)-1H-2,3-dihydro-1,4-benzodiazepines of the general formula I

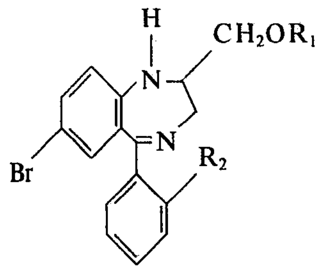

(I)

in which $R_1$ is hydrogen or alkyl with 1–3 carbon atoms or alkanoyl with 2–4 carbon atoms, and $R_2$ is halogen, and their acid addition salts, characterised in that 2-halomethyl-7-bromo-5-(2-halophenyl)-1H-2,3-dihydro-1,4-benzodiazepine compounds of the general formula III

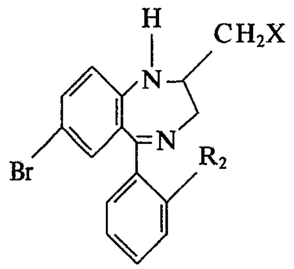

(III)

in which $R_2$ has the above meaning, and X is chlorine, bromine or iodine, or mixtures thereof, are cyclized in an organic solvent which is inert under the reaction conditions by treatment with a strong base to form 7-bromo-1,2-methylene-5-(2-halophenyl)-1H-2,3-dihydro-1,4-benzodiazepines of formula II

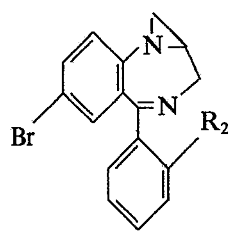

(II)

in which $R_2$ has the above meaning, and the compounds of formula II are reacted in the presence of a Lewis acid with compounds of formula IV

$$R_1'—OH \qquad (IV)$$

in which $R_1'$ is alkyl with 1–3 carbon atoms or alkanoyl with 2–4 carbon atoms to form compounds of formula Ia

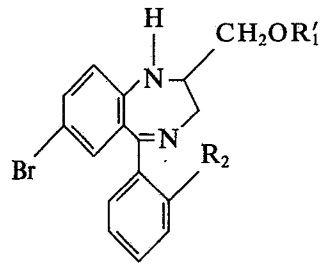

(Ia)

in which $R_1'$ and $R_2$ have the above meanings, and resulting esters of the general formula Ia, in which $R_1'$ is alkanoyl with 2–4 carbon atoms optionally are hydrolysed to form compounds of the general formula Ib

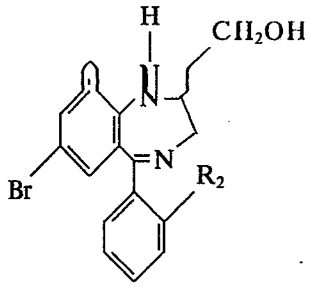

(Ib)

in which $R_2$ has the above meaning, and the resulting compounds of formula I optionally are converted into their acid addition salts or the acid addition salts are converted into the free compounds of formula I.

2. Method according to Claim 1 characterized in that such compounds of formula I are prepared, in which $R_2$ is chlorine.

3. Method according to Claim 1 or 2, characterized in that such compounds of formula I are prepared, in which $R_1$ is hydrogen, methyl or ethyl.

4. Method for the preparation of 7-bromo-1,2-methylene-5-(2-halophenyl)-1H-2,3-dihydro-1,4-benzodiazepines of the general formula II

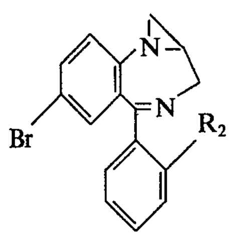

(II)

in which $R_2$ is halogen, and their acid addition salts characterized in that 2-halomethyl-7-bromo-5-(2-halophenyl)-1H-2,3-dihydro-1,4-benzodiazepine compounds of the general formula III

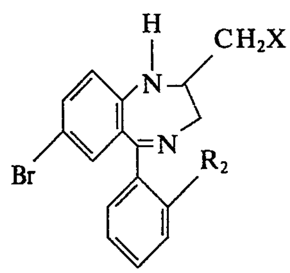

(III)

in which $R_2$ has the above meaning, and X is chlorine, bromine or iodine, or mixtures thereof are cyclized in an organic solvent which is inert under the reaction conditions by treatment with a strong base.

**Revendications pour les Etats contractants: BE, CH, DE, FR, GB, IT, LI, LU, NL, SE**

1. 7-bromo-5-(2-halogénophényl)-1H-2,3-dihydro-1,4-benzodiazépines de formule générale I.

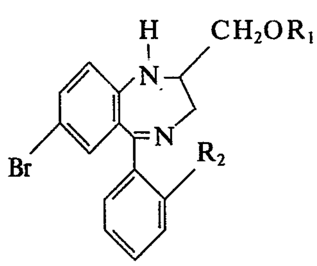

(I)

où $R_1$ est l'hydrogène, un radical alkyle ayant de l à 3 atomes de carbone ou alkanoyle ayant de 2 à 4 atomes de carbone, et $R_2$ est un halogène, et leurs sels d'addition d'acide.

2. Composés de formule I selon la revendication 1, où $R_2$ est le chlore.

3. Composés de formule I selon la revendication 1 ou 2, où $R_1$ est l'hydrogène, le radical méthyle ou éthyle.

4. 7-bromo-1,2-méthylène-5-(2-halogénophényl)-1H-2,3-dihydro-1,4-benzodiazépines de formule générale II

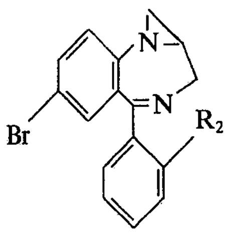

(II)

où $R_2$ est un halogène, et leurs sels d'addition d'acide.

5. Médicament contenant des composés de formule générale I selon la revendication I ou leurs sels d'addition d'acide pharmacologiquement acceptables, et des excipients pharmaceutiques courants.

6. Procédé pour la préparation des 7-bromo-5-(2-halogénophényl)-1H-2,3-dihydro-1,4-benzodiazépines de formule générale I

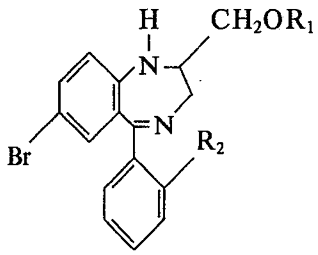

(I)

où $R_1$ est l'hydrogène ou un radical alkyle ayant de 1 à 3 atomes de carbone ou alkanoyle ayant de 2 à 4 atomes de carbone, et $R_2$ est un halogène, et de leurs sels d'addition d'acide, caractérisé en ce qu'on cyclise des 2-halogénométhyl-7-bromo-5-(2-halogénophényl)-1H-2,3-dihydro-1,4-benzodiazépine de formule générale III

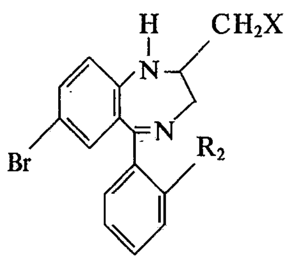

(III)

dans laquelle $R_2$ a la signification ci-dessus, et X est le chlore, le brome ou l'iode, ou leurs mélanges, dans un solvant organique inerte dans les conditions de la réaction par traitement avec une base forte, pour donner des 7-bromo-1,2-méthylène-5-(2-halogénophényl)-1H-2,3-dihydro-1,4-benzodiazépines de formule II

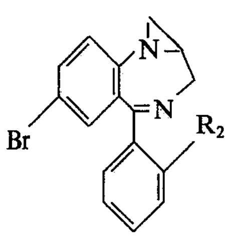

(II)

dans laquelle $R_2$ a la signification ci-dessus, et qu'on fait réagir les composés de formule II, en présence d'un acide de Lewis, avec des composés de formule IV

$$R_1'{-}OH \qquad (IV)$$

dans laquelle $R'_1$ est un radical alkyle ayant de 1 à 3 atomes de carbone ou alkanoyle ayant de 2 à 4 atomes de carbone, pour obtenir des composés de formule Ia

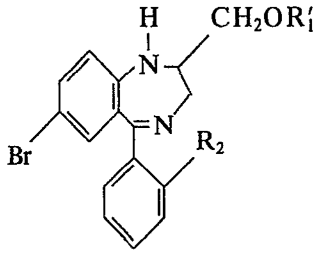

(Ia)

dans laquelle $R'_1$ et $R_2$ ont la signification ci-dessus, et qu'on hydrolyse éventuellement les esters obtenus de formule générale Ia, dans laquelle $R'_1$ est un radical alkanoyle ayant de 2 à 4 atomes de carbone, pour donner des composés de formule générale Ib

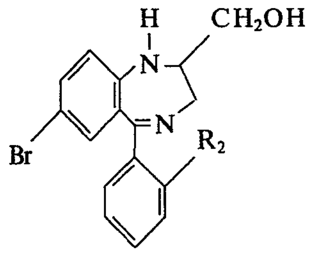

(Ib)

dans laquelle $R_2$ a la signification ci-dessus, et que, si on le souhaite, on convertit les composés obtenus de formule I en leurs sels d'addition d'acide ou les sels d'addition d'acide en composés libres de formule I.

**Revendications pour l'Etat contractant: AT**

1. Procédé pour la préparation des 7-bromo-5-(2-halogénophényl)-1H-2,3-dihydro-1,4-benzodiazépines de formule générale I

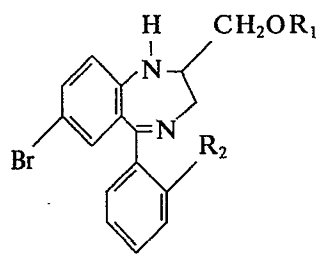

(I)

dans laquelle $R_1$ est l'hydrogène ou un radical alkyle de 1 à 3 atomes de carbone, ou alkanoyle ayant de 2 à 4 atomes de carbone, et $R_2$ est un halogène, et de leurs sels d'addition d'acide, caractérisé en ce qu'on cyclise des 2-halogénométhyl-7-bromo-5-(2-halogénophényl)-1H-2,3-dihydro-1,4-benzodiazépines de formule générale III

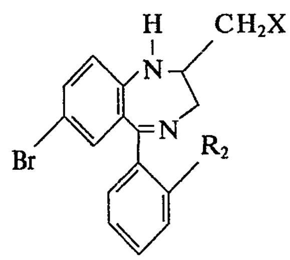

(III)

dans laquelle $R_2$ a la signification ci-dessus, et X est le chlore, le brome ou l'iode, ou leurs mélanges, dans un solvant organique inerte dans les conditions de la réaction par traitement avec une base forte

pour donner des 7-bromo-1,2-méthylène-5-(2-halogénophényl)-1H-2,3-dihydro-1,4-benzodiazépines de formule II

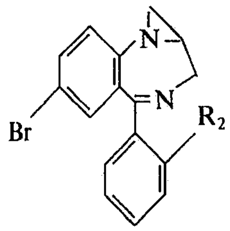

(II)

dans laquelle $R_2$ a la signification ci-dessus, et qu'on fait réagir les composés de formule II, en présence d'un acide de Lewis, avec des composés de formule IV

$$R_1'—OH \qquad (IV)$$

dans laquelle $R_1'$ est un radical alkyle ayant de 1 à 3 atomes de carbone ou alkanoyle ayant de 2 à 4 atomes de carbone, pour obtenir des composés de formule Ia

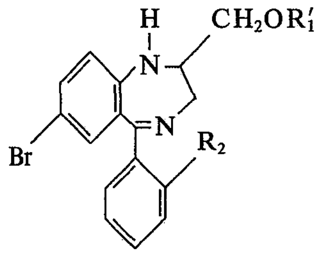

(Ia)

dans laquelle $R_1'$ et $R_2$ ont la signification ci-dessus, et qu'on hydrolyse éventuellement les esters obtenus de formule générale Ia, dans laquelle $R_1'$ est un radical alkanoyle ayant de 2 à 4 atomes de carbone, pour donner des composés de formule générale Ib

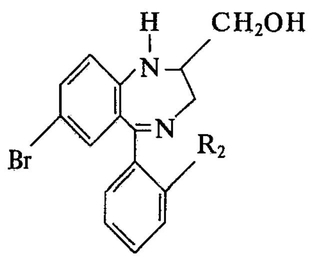

(Ib)

dans laquelle $R_2$ a la signification ci-dessus, et que, si on le souhaite, on convertit les composés obtenus de formule I en leurs sels d'addition d'acide ou les sels d'addition d'acide en composés libres de formule I.

2. Procédé selon la revendication 1, caractérisé en ce qu'on prépare des composés de formule I dans laquelle $R_2$ est le chlore.

3. Procédé selon la revendication 1 ou 2, caractérisé en ce qu'on prépare des composés de formule I dans laquelle $R_1$ est l'hydrogène, un radical méthyle ou éthyle.

4. Procédé pour la préparation de 7-bromo-1,2-méthylène-5-(2-halogénophényl)-1H-2,3-dihydro-1,4-benzodiazépines de formule générale II

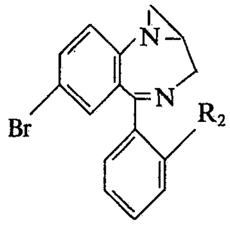

(II)

dans laquelle $R_2$ est un halogène, et de leurs sels d'addition d'acide caractérisé en ce qu'on cyclise des 2-halogénométhyl-7-bromo-5-(2-halogénophényl)-1H-2,3-dihydro-1,4-benzodiazépines de formule générale III

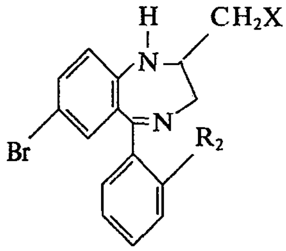

(III)

dans laquelle $R_2$ a la signification ci-dessus, et X est le chlore, le brome ou l'iode, ou leurs mélanges, dans un solvant organique inerte dans les conditions de la réaction par traitement avec une base forte.